Europäisches Patentamt

European Patent Office

Office européen des brevets

(19)

(11) Numéro de publication : **0 296 078 B1**

# FASCICULE DE BREVET EUROPEEN

(12)

(45) Date de publication du fascicule du brevet :
**29.05.91 Bulletin 91/22**

(51) Int. Cl.$^5$ : **A61L 31/00**

(21) Numéro de dépôt : **88420194.8**

(22) Date de dépôt : **14.06.88**

(54) **Nouveaux biomatériaux à base de mélanges de collagène, de chitosan et de glycosaminoglycanes, leur procédé de préparation ainsi que leurs applications en médecine humaine.**

(30) Priorité : **15.06.87 FR 8708752**

(43) Date de publication de la demande :
**21.12.88 Bulletin 88/51**

(45) Mention de la délivrance du brevet :
**29.05.91 Bulletin 91/22**

(84) Etats contractants désignés :
**AT BE CH DE ES FR GB GR IT LI LU NL SE**

(56) Documents cités :
**EP-A- 0 038 628**
**EP-A- 0 200 574**

(73) Titulaire : **CENTRE NATIONAL DE LA RECHERCHE SCIENTIFIQUE (CNRS)**
**15, Quai Anatole France**
**F-75007 Paris (FR)**

(72) Inventeur : **Collombel, Christian**
**34 rue de Trion**
**F-69005 Lyon (FR)**
Inventeur : **Damour, Odile**
**3 rue Charles Luizet**
**F-69230 Saint-Genis-Laval (FR)**
Inventeur : **Gagnieu, Christian**
**10 rue du Docteur Victor Despeignes**
**F-69008 Lyon (FR)**
Inventeur : **Poinsignon, Frédérique**
**1 rue du Lac**
**F-69003 Lyon (FR)**
Inventeur : **Echinard, Christian**
**Mas de la Baume**
**F-13420 Gemenos (FR)**
Inventeur : **Marichy, Jacques**
**2 rue des Balmes**
**F-69009 Lyon (FR)**

(74) Mandataire : **Maureau, Philippe et al**
**Cabinet Germain & Maureau Le Britannia - Tour C 20, bld Eugène Déruelle Boîte Postale 3011**
**F-69392 Lyon Cédex 03 (FR)**

## Description

La présente invention concerne de nouveaux biomatériaux à base de mélanges de collagène, de chitosan et de glycosaminoglycanes, leur procédé de préparation ainsi que leurs applications notamment en dermatologie pour le recouvrement des brûlés et en chirurgie plastique.

Selon l'invention, ces nouveaux biomatériaux sont caractérisés en ce qu'ils comprennent au moins un composé constitué par une association de collagène, de chitosan et de glycosaminoglycanes.

Le chitosan, de préférence obtenu par simple désacétylation de la chitine, reste acétylé à un degré d'acétylation compris entre environ 10% et environ 40%.

Les biomatériaux selon l'invention peuvent être utilisés dans toutes les pertes de substance et pour la reconstitution de tous les tissus.

En effet, les caractéristiques physiques (résistance, élasticité, taille des pores, temps de dégradation...) peuvent être modifiées et adaptées à l'utilisation prévue en jouant sur la proportion des différents constituants.

Une fois implantée, la matrice extracellulaire que constitue ce biomatériau sera colonisée par les cellules adéquates qui reconstitueront in vivo le tissu qui leur est spécifique.

C'est ainsi que les ostéoblastes vont synthétiser un tissu conjonctif qui se calcifiera pour donner de l'os. Les fibroblastes donneront des tissus conjonctifs (dermes, artères...) et les cellules nerveuses des nerfs.

La présente invention va d'abord être décrite dans une de ses applications très importantes qui est une peau artificielle permettant la reconstitution simultanée du derme et de l'épiderme tout en évitant la formation de cicatrices hypertrophiques.

Cette peau artificielle, entièrement biodégradable, trouvera ses principales applications pour le recouvrement précoce des brûlés après excision précoce des tissus nécrosés, ainsi que pour le traitement des plaies cutanées de l'homme et des animaux.

On sait qu'en cas de brûlures profondes et étendues, il est indispensable de reconstruire le plus rapidement et le plus durablement possible une barrière cutanée imperméable, d'une part, pour protéger les brûlés des infections et des pertes calorico-azotées, d'autre part, le derme sert de moule pour éviter les cicatrices hypertrophiques et les rétractions dues à la prolifération anarchique des fibroblastes.

Les homogreffes sont coûteuses et ne constituent pas une couverture efficace, car elles sont très souvent rejetées, ce qui entraîne un choc supplémentaire pour les malades.

Les techniques classiques d'autogreffe ne sont, par ailleurs, réalisables que dans le cas de brûlures moyennement étendues.

Les progrès de la réanimation médico-chirurgicale permettent, à l'heure actuelle, de maintenir en vie des brûlés atteints à 80% de la surface cutanée. Ces progrès ont eu comme conséquence d'accroître les besoins en peau ou équivalent de peau.

De nombreuses recherches ont été entreprises dans ce but.

C'est ainsi que Green et O'Connor se sont donné pour but de reconstituer l'épiderme à partir de cultures de kératinocytes provenant des propres cellules du malade.

Bell et son équipe ont, par ailleurs, mis au point un équivalent de peau constitué d'une couche dermique de fibroblastes autologues et de collagène bovin et d'une couche épidermique de kératinocytes autologues.

Il convient de noter que la mise en oeuvre de ces deux techniques nécessite un temps d'attente de vingt-et-un jours (correspondant à la durée de culture des cellules) avant de pouvoir disposer de la peau artificielle convenant au patient.

Une troisième technique a été mise au point par Yannas et Burke (Jal of Biomedical Materials Research-Vol 14, 65-81, 107-131, 511-528 (1980) et FR-A-2 332 863).

La peau artificielle proposée par ces auteurs comprend deux couches :
– un équivalent dermique constitué de collagène de veau et d'un glycosaminoglycane (GAG), la chondroïtine 6-sulfate, et réticulé par du glutaraldehyde ;
– un pseudo-épiderme temporaire en résine de silicone, non-biodégradable limitant les pertes exudatives de la phase précoce et faisant office de barrière contre les agents infectieux, venant recouvrir la couche dermique.

Cette peau artificielle est mise en place après excision précoce du tissu brûlé au troisième degré (48 à 72 heures après la brûlure). Le réseau collagène-GAG contient des pores à travers lesquels le néoderme endogène est en quelque sorte "canalisé". Ainsi est reconstitué un tissu cicatriciel "domestiqué", très proche du derme normal, et au niveau duquel la synthèse de collagène par les fibroblastes ne se fait pas de façon anarchique à la différence du tissu de granulation habituel. Lorsque la couche dermique est revascularisée (3 à 4 semaines), et lorsque l'on dispose de cellules épidermiques, la membrane de silicone est enlevée. On peut alors mettre en place soit une greffe épidermique extrêmement mince, en filet 4 ou 9, (prélevée sur les zones donneuses restantes).

Ce procédé de recouvrement cutané après excision précoce, déjà testé aux USA sur plus de deux cents brûlés, dans cinq centres, s'avère particulièrement efficace, tant pour combattre les pertes exudatives hydriques et calorico-azotées de la phase précoce, que pour prévenir l'infection et pour diminuer l'importance des séquelles hypertrophiques et rétractiles.

Cette matrice, compacte et réticulée, est colonisée par les fibroblastes du blessé qui synthétisent leur propre collagène et toutes les protéines humaines nécessaires à la reconstitution du tissu conjonctif.

Le temps de dégradation du derme artificiel, qui est de vingt-cinq jours, correspond au temps de cicatrisation de la plaie.

Ainsi canalisés, les fibroblastes du brûlé peuvent prendre une bonne orientation, ce qui évite les cicatrices hypertrophiques.

Il reste naturellement à épidermiser dans un second temps.

Ce matériau répond aux besoins d'urgence. Il peut être stérilisé et stocké en grande quantité.

Cette technique, pour intéressante qu'elle soit, présente pourtant plusieurs inconvénients, tant en ce qui concerne le derme, qu'en ce qui concerne le pseudo-épiderme.

## Pour le derme :

L'association du collagène et des glycosaminoglycanes décrite par YANNAS et BURKE donne des matériaux composites dans lesquels les constituants sont aisément dissociables en raison notamment de la pauvreté du collagène en fonctions basiques ionisables. Ces matériaux composites sont solubles dans les liquides de l'organisme et doivent être réticulés au niveau du collagène pour que soit possible leur utilisation en milieu aqueux. Cependant, la faible cohésion entre le collagène réticulé et les glycosaminoglycanes acides est responsable d'un relargage progressif des GAG à chaque fois qu'une immersion dans une solution aqueuse est nécessaire (hydratation, lavage, neutralisation, etc...). Le relargage, qu'il est difficile de contrôler, modifie de façon importante la composition finale des dermes.

Pour diminuer sa dégradation, augmenter sa résistance, il est nécessaire de réticuler le coprécipité de collagène-GAG, soit par réticulation chimique utilisant le glutaraldéhyde, produit toxique pour l'organisme, ce qui nécessite, d'une part, d'éliminer le glutaraldéhyde en excès et, d'autre part, de contrôler son élimination du produit fini, soit par réticulation physique par action simultanée de la chaleur et du vide.

## Pour le pseudo-épiderme :

Le pseudo-épiderme en résine de silicone n'est pas biodégradable. Il est donc nécessaire de l'enlever chirurgicalement avant d'épidermiser.

Il est, par ailleurs, inutilisable comme support de culture in-vitro et in-vivo.

La dernière technique en date est celle proposé par WIDRA (EP-A-0 089 152 et EP-A-0 138 385), qui propose une peau artificielle réalisée à partir de l'association de composés anioniques dérivés de la kératine et de biopolymères cationiques tels que le chitosan et le collagène.

Ces composés sont applicables en deux étapes pour former, in situ, une membrane hydrogel qui forme une carapace protectrice et, en fin de cicatrisation, durcit et tombe.

Dans la présente invention, les Inventeurs se sont donné pour but de préparer une nouvelle peau artificielle ne présentant pas les défauts des techniques mentionnées ci-avant. Dans ce but, ils se sont fixé certains critères auxquels devront satisfaire le derme et l'épiderme.

Le derme devra, bien entendu :
– faciliter la migration des fibroblastes et des cellules endothéliales ;
– ne présenter que peu ou pas d'antigénicité,
– se dégrader à une vitesse contrôlable,
– permettre de réduire et de prévenir les contractures,
– activer la cicatrisation et prévenir l'infection,
– ne pas provoquer de réaction inflammatoire,
– diminuer la douleur,
– prévenir les cicatrices hypertrophiques.

Il devra, en outre, être bien toléré, être résistant et manipulable sans qu'il soit nécessaire de recourir à une réticulation des constituants.

L'épiderme devra être entièrement biodégradable et susceptible de servir de support de culture cellulaire; il devra, en outre, être imperméable aux bactéries, réguler le flux liquidien et présenter des pores de dimensions contrôlées.

La peau artificielle selon l'invention est caractérisée en ce qu'elle est essentiellement constituée d'une couche dermique de type velours comprenant l'association de collagènes, de chitosan, de chondroïtines 6 et 4 sulfate et éventuellement d'acide hyaluronique. Cette couche dermique est associée à un pseudo-épiderme biodégradable à structure membranée.

La composition de la couche dermique selon l'invention est tout à fait originale. On sait en effet que l'addition de molécules polyanioniques ou polycationiques à du collagène conduit à la formation de réseaux ioniques dans lesquels interviennent soit les groupements amine soit les groupements carboxyle de cette protéine. La densité de ces liaisons, dans les matériaux composites obtenus dépend de l'accessibilité de ces groupements, de leur répartition et de leur nombre.

Il existe déjà des complexes associant le collagène et le chitosan. C'est ainsi que KOSUGI et KOTTO (US-A-4378017) préparent des composites collagène bovin fibreux-chitosan macrofibrillaire de crabes utilisable en industrie alimentaire. Il s'agit d'un acétyl-chitosan dont le degré d'acétylation est de préférence compris entre 0 et 30%. Ce matériau fait intervenir des liaisons ioniques entre ses constituants mais celles-ci apparaissent comme totalement insuffisantes pour garantir sa stabilité et sa solidité. Il est indispensable de recourir à une réticulation chimique pour stabiliser le complexe. Les membranes obtenues par les auteurs sont transparentes, très résistantes à la traction, élastiques et thermostables. Elles sont de plus insolubles dans l'eau et en milieu acide contrairement au collagène et au chitosan de départ pris séparément. Ces membranes sont cependant hydratables en milieu aqueux sans qu'il y ait relargage des constituants et donnent des structures souples et homogènes dont les propriétés physicochimiques et biologiques sont compatibles avec leur utilisation éventuelle comme support de cultures cellulaires.

Dans ce cas, seuls les groupements carboxyles du collagène interviennent dans la formation du réseau.

EP-A-200 574 décrit un biomatériau composé d'atélocollagène, de N-acyl chitosan et éventuellement de chondroïtines-sulfate.

Le N-acyl-chitosan décrit dans ce document est obtenu par un procédé en deux étapes. La première de ces étapes consiste en une désacétylation à au moins 55%, et de préférence de 70 à 100% de la chitine. La deuxième étape consiste en une réacylation du chitosan obtenu, notamment avec de l'acide acétique ou succinique, jusqu'à un degré d'acylation égal à au moins 55%, mais de préférence compris entre 70 et 100%.

Cet acylchitosan est acylé à un degré d'acylation au moins égal à 55% mais de préférence compris entre 70 et 100%.

Or, une acylation à au moins 55% mais de préférence comprise entre 70 et 100% signifie que l'acylchitosan présente, au maximum, 45% de fonctions amines $NH_3^+$ libres. Cela limite les possibilités de liaison de ces fonctions amines avec des groupements $COO^-$ du collagène ou des groupements $SO_4^-$ et $COO^-$ des chondroïtines-sulfate, selon le schéma suivant :

Acylchitosan acylé avec de l'acide acétique

$$\begin{array}{ccccc} NH_3^+ & NH\text{-}CO\text{-}CH_3 & NH\text{-}CO\text{-}CH_3 & NH_3^+ & NH\text{-}COC \\ | & & & | & \\ | & & & | & \\ COO^-\text{-collagène} & & & COO^-\text{- chondroïtines} & \\ & & & \text{sulfate} & \end{array}$$

Le nombre de liaisons ioniques entre constituants est donc relativement faible et le réseau ionique est beaucoup moins important.

Ainsi, comme le précise EP-A-200 574, il est préférable d'avoir recours à un procédé de réticulation chimique (utilisation d'hexaméthylène diisocyanate ou de glutaraldéhyde), afin d'augmenter, de façon suffisante, la résistance et la capacité d'absorption en eau et d'empêcher le relargage des glycosaminoglycanes.

Dans le cas où l'acylation s'effectue avec un diacide comme l'acide succinique, cela entraîne une augmentation du nombre de fonctions carboxyliques $COO^-$ qui se lient, de façon intramoléculaire, avec les fonctions amines $NH_3^+$ du succinylchitosan lui-même et de façon intermoléculaire avec les fonctions amines $NH_3^+$ d'une autre chaîne de succinylchitosan ou du collagène.

Il reste donc encore moins de fonctions amines $NH_3^+$ libres aptes à se lier avec des groupements acides du collagène et/ou des glycosaminoglycanes. On a :

4

$$\begin{array}{ccccc}
NH_3{}^+ & NH\text{-}CO\text{-}Succ\text{-}COO^- & NH_3{}^+ & NH\text{-}Co\text{-}Succ\text{-}COO^- & \\
| & & & & \\
| & & & & \\
| & & & Chitosan\text{-}NH_3{}^+\ OU\ NH_3{}^+ & \\
Collag\grave{e}ne\text{-}COO^- & & & | & \\
& & & C\acute{o}llag\grave{e}ne &
\end{array}$$

Par ailleurs, le biomatériau obtenu selon l'enseignement de ce document doit être réticulé chimiquement afin d'être de qualité correcte.

Les inventeurs ont découvert qu'il était possible d'obtenir des résultats tout à fait exceptionnels en formant des réseaux ioniques dans lesquels interviennent à la fois les groupements carboxyle et les groupements amine du collagène.

La participation simultanée de ces deux groupements ionisables est obtenue en associant le collagène à la fois aux chondroïtines sulfate et au chitosan. La structure obtenue est stabilisée par la formation de liaisons ioniques entre les fonctions acides des chondroïtine sulfate et les fonctions amine du chitosan.

Cette association permet la formation de réseaux ioniques dans lesquels interviennent à la fois les groupements carboxyle et amine du collagène qui se lient respectivement aux fonctions amine du chitosan présentant un faible taux d'acétylation résiduelle et aux groupements sulfate et carboxyle des chondroïtines 4 et 6-sulfate. En outre, les groupements acide et sulfate des chondroïtines 4 et 6-sulfate peuvent se lier ioniquement aux fonctions amine du chitosan.

La cohésion de ces réseaux ioniques est suffisamment forte pour que le derme obtenu soit insoluble en milieu aqueux et pour qu'il n'existe pas de relargage des chondroïtines-sulfates. Cette stabilité qui se traduit en particulier par l'absence de relargage des chondroitines sulfate permet d'éviter l'utilisation des procédés habituels de réticulation par des liaisons covalentes (glutaraldéhyde, azide d'acide, diisocyanates...).

Le constituant majoritaire de la couche dermique selon l'invention est le collagène, et de préférence le collagène type I + III, natif ou sans télopeptides à structure hélicoïdale préservée, qu'il s'agisse de collagène de provenance humaine, extrait du placenta, ou de collagène bovin. Ce collagène soluble dans les fluides de l'organisme est entièrement dégradable par les enzymes cellulaires et est parfaitement toléré par l'organisme humain.

Le chitosan (polyglucosamine) est le deuxième composant du derme selon l' invention. Ce chitosan peut être de différentes provenances.

Comme mentioné ci-avant, il est obtenu en une seule étape par N-désacétylation de chitine (poly-N-acétyl-glucosamine) microfibrillaire de carapaces de crevettes. Ce polyoside aminé présente un haut pouvoir d'absorption des protéines riches en acides aminés acides (acides glutamique et aspartique).

La vitesse de dégradation enzymatique du chitosan est fonction à la fois de sa masse moléculaire de son degré d'acétylation. Le chitosan entrant dans la composition des dermes, objet de l'invention, présente un poids moléculaire moyen compris entre 150.000 et 1.000.000 et des degrés d'acétylation compris entre 40 et 10%. Ce taux d'acétylation constitue une différence essentielle avec les matériaux de la technique antérieure décrite ci-avant ; en effet, l'acétylchitosan selon l' invention présente suffisamment de groupements amines libres pour se lier à la fois au collagène et aux glycosaminoglycanes. Les variations dans les poids moléculaires et les degrés d'acétylation permettent de modifier la cohésion des constituants des dermes et de moduler ainsi leurs propriétés biologiques et physicochimiques en fonction des buts recherchés.

Par ailleurs, le chitosan présente des propriétés biologiques intéressantes utilisables en clinique. Il est hémostatique et cicatrisant et peut être utilisé comme support de culture cellulaire. Son rôle de protection de l'organisme animal vis-à-vis des infections bactériennes et fongiques a été également démontré. Il agit par stimulation du système immunitaire et, en particulier, il induit l'activation des macrophages. Cette propriété a été utilisée chez l'animal dans le traitement des tumeurs cancéreuses.

Le chitosan et le composite collagène-chitosan présentent donc des propriété physicochimiques et biologiques compatibles avec leur utilisation comme constituant des dermes artificiels.

Les 3ème et 4ème constituants, les chondroïtines sulfate et l'acide hyaluronique sont des glycosaminoglycanes.

Les glycosaminoglycanes font partie intégrante du tissu conjonctif.

5

Les glycosaminoglycanes sont caractérisé par des unités disaccharidiques linéaires, généralement constitués d'un acide uronique (acide glycuronique ou acide iduronique et d'une hexosamine (glucosamine ou galactosamine) très souvent N-acétylée et estérifiée par l'acide sulfurique.

Les principaux glycosaminoglycanes sont :
– l'acide hyaluronique,
– la chondroïtine 4-sulfate ou chondroïtine sulfate A (CSA),
– la chondroïtine 6-sulfate ou chondroïtine sulfate C (CSC),
– le dermatane sulfate ou chondroïtine sulfate B (CSB),
– l'héparane sulfate (HS),
– le kératane sulfate (KS) qui diffère des autres glycosaminoglycanes par la présence de galactose à la place de l'acide uronique.

Les chondroïtines 4- et 6-sulfate sont les glycosaminoglycanes les plus répandus. Ce sont des polymères dont l'unité fondamentale est la suivante : glucuronyl ($\beta$ 1.3)N-acétylgalactosamine sulfate ($\beta$ 1.4). Les chondroïtines 4- et 6-sulfate ne diffèrent que par la position du groupement sulfate. La molécule de galactosamine est estérifiée soit en position 4 (chondroïtine 4-sulfate ou chondroïtine sulfate A), soit en position 6 (chondroïtine 6-sulfate ou chondroïtine sulfate C).

On a démontré que les glycosaminoglycanes sulfatés, à l'exception du kératane sulfate, pouvaient former, in vitro et à des pH physiologiques, des liaisons ioniques avec le collagène. Ces liaisons sont d'autant plus fortes que le contenu en acide iduronique du glycosaminoglycane est élevé.

La co-précipitation du collagène et des chondroïtine 6-sulfate à pH acide ralentit la dégradation du collagène en masquant la site d'action de la collagènase et diminue son immunogénicité en masquant les sites antigéniques. Les chondroïtines sulfate rendent le collagène hémocompatible en diminuant l'agrégation des plaquettes et la libération de sérotonine.

Les chondroïtines 6 et 4-sulfate utilisées dans la présente invention, en même temps que le chitosan, comme précisé ci-avant, sont avantageusement associés à l'acide hyaluronique précédemment décrit qui exerce un rôle de régulation de la croissance et de la migration cellulaire. L'acide hyaluronique est le seul glycosaminoglycane non sulfaté.

Il s'agit d'un polymère d'unité : glucuronyl ($\beta$ 1,3) N- acétyloglucosamine ($\beta$ 1,4). L'acide hyaluronique a un poids moléculaire variant entre 100.000 et plusieurs millions selon le tissu considéré.

Il faut souligner que l'introduction, dans des formulations à base de collagène et de chondroïtines sulfate, de chitosan, outre les améliorations qu'elle communique au derme sur le plan biologique, apporte une solution au problème du relargage et de la réticulation. En effet, ce polysaccharide présente de très nombreuses fonctions aminées ionisables susceptibles de se lier par liaison ionique à chacun des constituants du derme puisqu'ils présentent tous des fonctions acides (carboxylique et sulfonique) en proportion importante.

Dans ce cas, et contrairement à ce qui se passe après réticulation chimique du collagène seul dans les mélanges collagène-GAG connus, tous les constituants sont liés entre eux par un nombre de liaisons ioniques suffisamment important pour que le relargage soit négligeable. La composition chimique des dermes obtenus reste invariable au cours des opérations nécessitant une immersion dans des solutions aqueuses. La présence de chitosan dans les équivalents dermiques selon l'invention permet donc d'obtenir un matériau de composition très définie et qui présente la particularité d'être insoluble dans les fluides de l'organisme sans qu'il soit nécessaire de recourir à une réticulation chimique covalente.

Par ailleurs, la couche pseudo-épidermique recouvrant les dermes artificiels doit avoir des propriétés physicochimiques proches des épidermes naturels. Cette couche doit agir comme régulateur du flux hydrique et comme barrière vis-à-vis des agents infectieux. Elle doit, en outre, par ses propriétés mécaniques, conférer à l'ensemble derme-épiderme une solidité et une élasticité compatibles avec les conditions de manipulation intervenant au moment de la greffe (hydratation, lavage, découpage, suture...).

Selon l'invention, cette couche est constituée par un film de chitosan seul ou associé à des chondroïtines-sulfate et/ou du collagène. Elle peut être constituée également de ces éléments seuls ou associés à des protéines de structure modifiées telles que les carboxyméthyl ou aminoéthyl-kératéines substituées.

La couche dermique comporte entre 10 et 20% de chitosan, entre 4 et 10% de chondroïtines 4- et 6- sulfate, ces pourcentages étant calculés par rapport au poids du collagène.

L'utilisation d'autres polyélectrolytes en association avec une ou plusieurs de ces macromolécules ionisables est possible. En particulier les polyosides végétaux (acide polygalacturonique...) ou les poly-aminoacides peuvent être employés pour la constitution de réseaux ioniques présentant les propriétés physicochimiques désirées.

Ces films composites sont rendus plus ou moins lipophiles par greffage chimique de substituants non toxiques tels que les acides gras ou d'autres substances hydrophobes, ou par application superficielle d'un succédané de sébum contenant des acides gras libres ou estérifiés ou toute autre substance contenue dans le

6

sébum.

Les pseudo-épidermes selon l'invention présentant l'avantage, par rapport aux substances chimiques généralement utilisées pour l'imperméabilisation des dermes, présentent une lipophilie modulable en fonction de la nature des substances grasses utilisées ou de la nature des substituants lipophiles introduits.

La présente invention sera mieux comprise et ses avantages ressortiront bien des exemples suivants.

Dans tous les exemples, le chitosan est obtenu par N-désacétylation de chitine (poly-N-acétylglucosamine) microfibrillaire provenant de carapaces de crevettes. Il s'agit d'un chitosan dont le degré d'acétylation se situe entre environ 10 et environ 40%.

## EXAMPLE 1 : PREPARATION DU DERME COMPOSANT L'ESSENTIEL DE LA PEAU ARTIFICIELLE

Préparation de la solution de collagène :

Les collagènes préférés appartiennent au type III + I, au type III, au type I, au type IV et au type V et se trouvent sous forme natifs ou sans télopeptides, à structure hélicoïdale préservée, en solution ou sous forme fibreuse.

Ils sont préparés, de façon classique, à partir de derme de veau (collagènes commercialisés en France par la S.A. de Développement et d'Utilisation du Cuir). Ils peuvent également être obtenus à partir de placenta humain (collagènes commercialisés en France par l'Institut Mérieux).

La solution est réalisée en mélangeant à l'aide d'un agitateur 1% de collagène (poids/volume) dans de l'acide acétique 0,05 M à température ambiante et pH 3,5.

Si l'on désire un derme artificiel moins rigide, la concentration de la solution peut être abaissée jusqu'à 0,3% (poids/volume).

### Addition du chitosan :

On prépare le chitosan par N-désacétylation de chitine (poly-N-acétyl-glucosamine) microfibrillaire provenant de carapaces de crevettes.

– Le chitosan ainsi obtenu et hautement purifié est ajouté à la solution précédente à raison de 15% (poids/poids) par rapport au collagène.

Le chitosan joue, en plus de ses propriétés naturelles, le rôle d'agent réticulant ionique. Son poids moléculaire peut varier de 150 000 à 1 000 000.

Le degré d'acétylation, que l'on peut faire varier de 40 à 10%, détermine le degré de réticulation, et permet donc de jouer sur la vitesse de dégradation du derme artificiel.

### Addition des chondroïtines sulfates

- Un mélange de chondroïtines 4 et 6 sulfate purifié à 99% à partir de cloisons nasales d'ovins est ensuite ajouté à la solution collagène-chitosan à raison de 6% (poids-poids) par rapport à la quantité de collagène initiale. La présence des deux premiers composés facilite la solubilisation des GAG qui se dissolvent rapidement sous agitation à température ambiante.

– On peut, à ce stade de la préparation, ajouter à la solution précédente de l'acide hyaluronique, à raison de 1% (poids-poids) par rapport au collagène. Ce produit permet une amélioration de l'adhésion cellulaire et permet également d'améliorer encore la croissance cellulaire, déjà favorisée par les glucosaminoglycanes.

### Neutralisation :

Une fois que l'on a obtenu un mélange bien homogène des différents constituants, on effectue la neutralisation du derme.

Le pH est alors ramené à 6,5-7 par une solution de NaOH N ou par un tampon Tris-HCl (pH 10,5).

Une autre méthode consiste à effectuer la neutralisation après lyophilisation de la solution. Il faut alors rincer le derme dans deux bains successifs d'eau stérile, de sérum physiologique ou de tampon Tris-HCl.

### Lyophilisation :

La solution homogène obtenue est lyophilisée dans des lyophilisateurs industriels.

L'épaisseur du derme dépend de la quantité de solution versée dans les moules et peut être adaptée aux besoins des utilisateurs. En moyenne, cette épaisseur varie de 1 à 8 mm selon l'utilisation.

On peut aussi réaliser des films à partir de la solution homogène précédemment préparée.

Cette solution est versée dans des moules et maintenue dans une étuve à 35°C pendant 48 heures, ce qui permet d'obtenir un derme artificiel lisse et transparent de 0,1 à 0,8 mm d'épaisseur.

## Stérilisation :

Deux principaux procédés de stérilisation sont retenus :
- l'alcool à 70°
- les rayons gamma.

Alcool à 70° :

Les dermes artificiels sont conditionnés dans des poches plastiques remplies d'alcool à 70° selon des techniques classiques évitant au maximum la contamination bactérienne ; tous les tests bactériologiques réalisés entre sept jours et six mois après le conditionnement dans l'alcool se sont révélés négatifs.

Rayonnement gamma :

Les dermes artificiels peuvent également être stérilisés par des irradiations ionisantes. Les rayons gamma proviennent d'une source de cobalt 60.

La dose de rayonnement peut s'étendre de 1 à 3 Megarad sans détérioration des propriétés du derme.

Le derme artificiel ainsi obtenu a été soumis à différents contrôles qui vont être exposés ci-après. Certains de ces contrôles sont représentés au dessin schématique annexé, dans lequel :

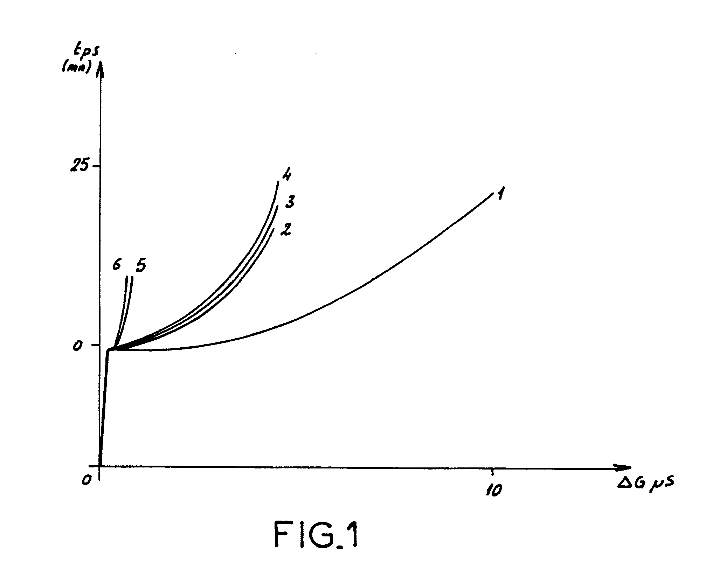

Figure 1 est une courbe représentant la vitesse de dégradation enzymatique du derme selon l'invention par la collagènase ;

Figure 2 représente les spectres de diffraction aux rayons X du collagène seul ;

Figure 3 représente le spectre de diffraction aux rayons X du derme artificiel selon l'invention, et d'un collagène dénaturé ;

Figure 4 représente les spectres de diffraction aux rayons X du derme artificiel selon l'invention une fois stérilisé aux rayons X et à l'alcool à 70° ;

Figure 5 représente les résultats d'électrophorèse sur un derme réalisé à partir de collagène bovin.

## Microscopie électronique :

La microscopie électronique à balayage permet d'observer la structure poreuse des dermes lyophilisés, la structure lamellaire des dermes filmogènes, et de contrôler la taille des pores déterminée par le pourcentage de matière sèche du derme.

La microscopie électronique à transmission permet de visualiser la structure microfibrillaire du derme selon l'invention.

## Propriétés mécaniques du derme artificiel :

Les tests de résistance des matériaux à la traction sont réalisés sur un appareil de mesure ADAMEL LHOMERGY DY 21b. Les échantillons sont préparés sous forme d'haltères de 0.5 cm de large sur 3 cm de long et 0,33 mm d'épaisseur. Chaque extrémité de l'éprouvette est pincée fortement entre les mors de l'appareil.

Les mesures d'étirement sont réalisées à une vitesse de 5 ou 10 mm/minute suivant les échantillons. Trois essais sont réalisés pour chaque mesure. Les résultats sont donnés dans le tableau I ci-après.

## TABLEAU I

| Echantillons | Stérilisation | Force da N | Allongement (mm) | Module de Young Kg/cm² |
|---|---|---|---|---|
| Collagène H | Non stérile | 0,01 | Très faible | / |
| Collagène + GAG | Non stérile | 0,02 | Très faible | / |
| Collagène H + Ch + GAG | Non stérile | 0,9 | 14,33 | 0,16 |
| Collagène H + Ch + GAG | Rayons Gamma | 0,1 | 17,3 | 0,14 |
| Collagène H + Ch + GAG | Alcool 70° | 0,09 | 15,7 | 0,145 |
| Collagène H + Ch + GAG + pseudo-épiderme bio-dégradable | Alcool 70° | 0,23 | 20,1 | 0,29 |
| Collagène B + Ch + GAG | Non stérile | 0,06 | 12,27 | 0,13 |
| Collagène B + Ch + GAG | Rayons Gamma | 0,07 | 13,00 | 0,13 |
| Collagène B + Ch + GAG | Alcool 70° | 0,06 | 13,3 | 0,12 |

Collagène H : Collagène humain

Collagène B : Collagène bovin

Ch : chitosan

GAG : glycosaminoglycanes.

La comparaison des échantillons constitués de collagène seul, de collagène et de glycosaminoglycanes et de collagène, chitosan et glycosaminoglycanes montre que la présence du chitosan augmente de façon importante la résistance mécanique du derme artificiel.

Comme on peut le voir, l'incorporation de 15% en poids de chitosan multiplie par quatre la force nécessaire pour obtenir la rupture du composé. Ceci montre clairement la forte cohésion des trois molécules entre elles.

**Etude de la vitesse de dégradation enzymatique du derme artificiel par la collagènase :**

Les mesures conductimétriques sont réalisées selon la technique suivante :

4 ml d'une suspension d'échantillon dans du tampon TrisCaCl$_2$ 1 mM pH 8 sont placés dans une cellule thermostatée à 30°C.

La conductance est enregistrée grâce à un pont de mesure à transformateur WAYNE-KERR B 641 dès que la stabilité thermique est atteinte (au bout de dix minutes environ). L'enzyme (collagénase de <u>Clostridium histolyticum</u> EC 34 243-Sigma Numéro C-0773) est alors introduite dans la cellule (concentration finale en enzyme : 3,24 U/ml) et la variation de conductance est enregistrée.

La réaction enzymatique est standardisée par addition d'un petit volume (50 µl) d'HCl 10 M. Les variations de conductance dues à l'hydrolyse sont alors converties en équivalent de protons libérés dans le milieu par la réaction enzymatique. Les vitesses d'hydrolyse s'expriment en équivalent de protons libérés par litre et par heure (H/l/h).

## TABLEAU II

| Echantillons | Stérilisation | % Activité enzymatique | Vitesse de dégradation en µm ($H^+$/l/h) |
|---|---|---|---|
| Collagène humain (1) | Non stérilisé | 100 | 1050 |
| Collagène H + Ch + GAG (2) | Non stérilisé | 44,8 | 462 |
| Collagène H + Ch + GAG (3) | Rayons Gamma | 32,2 | 402 |
| Collagène H + Ch + GAG (4) | Alcool 70° | 38,9 | 340 |
| GAG (5) | non stérile | 0 | 0 |
| Chitosan (6) | non stérile | 0 | 0 |

L'addition de chitosan et de GAG diminue de moitié la dégradation du collagène par la collagénase. Il n'y a pas de différence significative entre le derme artificiel non stérile et celui stérilisé aux rayons gamma ou à l'alcool à 70°.

## Etude de la diffraction aux rayons X

Cette étude permet de vérifier l'intégrité de la molécule de collagène après fabrication du derme artificiel et après stérilisation.

Les échantillons sont préparés à l'emporte-pièce et aplatis de façon à obtenir des pastilles de 1 cm de diamètre et 1 mm d'épaisseur. Ces pastilles sont placées dans un appareil de diffraction des rayons X relié à un système informatique.

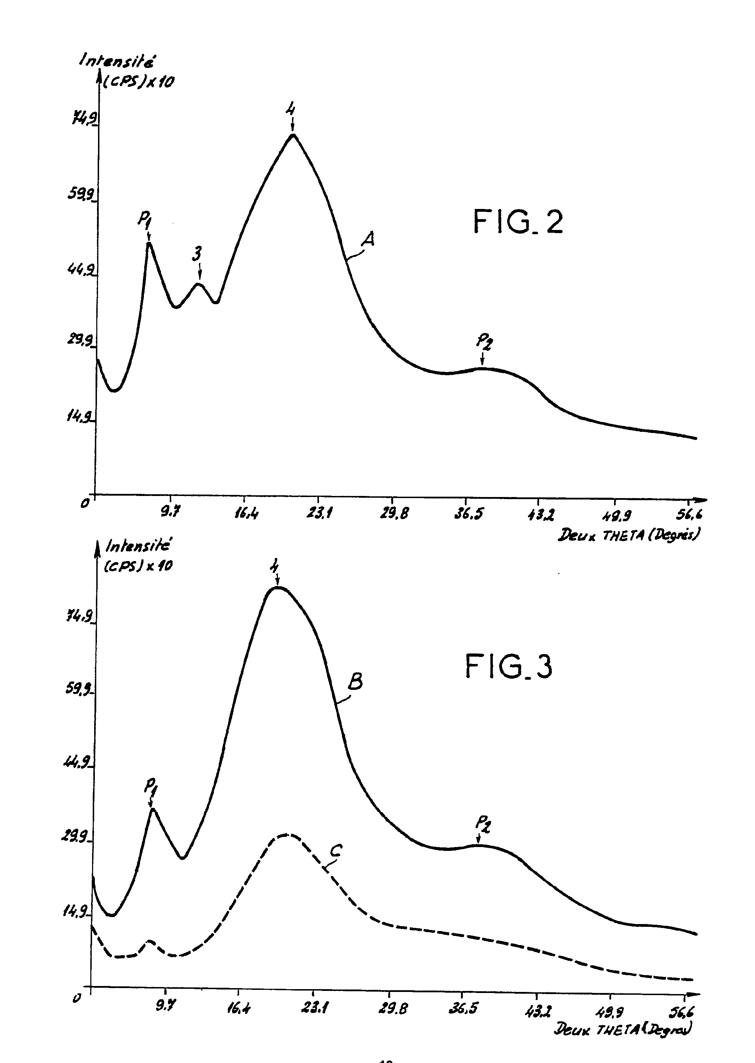

Les résultats sont donnés aux figures 2 à 4.

On peut observer les pics $P_1$ et $P_2$ caractéristiques du collagène sur la courbe A représentée à la figure 2.

Le pic $P_1$ permet de déterminer la distance entre les molécules de collagène. Le Pic $P_2$ traduit la distance entre deux acides aminés. C'est sa présence qui permet de déterminer le degré d'intégrité de la molécule de collagène.

Le pic 3 permet de connaître le pas des hélices de la molécule de collagène. C'est un pic particulièrement difficile à visualiser.

Le pic 4 n'est pas exploitable car il est donné par tous les éléments amorphes contenus dans le derme artificiel.

Sur la figure 3, la courbe B est celle du derme selon l'invention et la courbe C celle du collagène dénaturé. On peut observer que le procédé de fabrication n'altère pas la molécule de collagène, puisque le pic $P_2$ est toujours présent.

Le pic 3 n'est plus visible. Il doit être masqué par les résidus du chitosan et de glycosaminoglycanes.

Le pic $P_1$ conserve une amplitude identique dans le derme artificiel et dans du collagène seul.

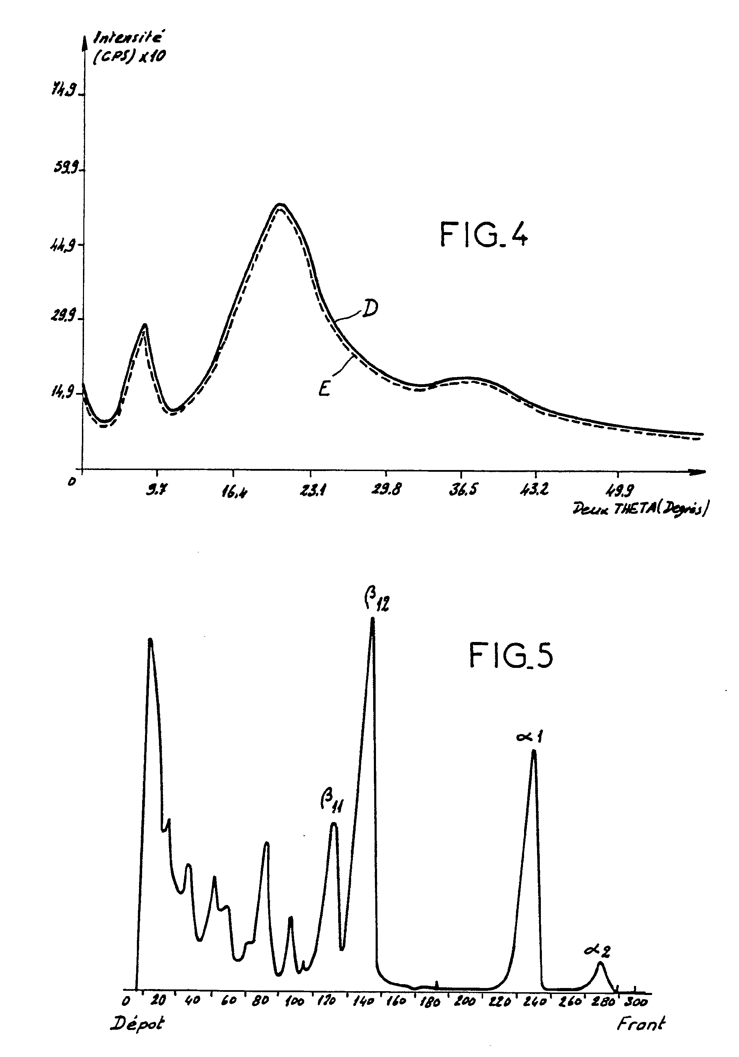

Les spectres de diffraction des rayons X observés sur la figure 4 montrent que les procédés de stérilisation n'altèrent pas non plus la molécule de collagène. Sur la figure 4, la courbe D est celle du derme stérilisé aux rayons X et la courbe E, celle du derme stérilisé à l'alcool à 70°.

## Electrophorèses

Une première série d'électrophorèses sur gel de polyacrylamide en présence de SDS a été réalisée. Elle permet de séparer les chaînes en fonction de leur poids moléculaire.

Les résultats d'électrophorèse du derme artificiel obtenu à partir de collagène bovin sont rassemblés à la figure 5. Les pics alpha$_1$, alpha$_2$, Bêta$_{11}$, Bêta$_{12}$, caractéristiques d'un collagène bovin acido-soluble, sont présents et sont tout à fait comparables à ceux obtenus avec un collagène bovin seul. L'absence de pic correspondant à des molécules de faible poids moléculaire confirmer que la fabrication n'altère donc pas les molécules de collagène, ce qu'avait déjà laissé supposer l'étude de la diffraction aux rayons X.

## Etude de la cytotoxicité

Une étude de cytotoxicité directe et indirecte du derme artificiel selon l'invention sur des cultures de fibroblastes et de kératinocytes a été réalisée. Les résultats sont rapportés dans le tableau III.

Dans chaque cas, aucun signe de cytotoxicité n'à été observé.

La morphologie des cellules reste identique à celle des témoins. La vitesse de croissance est légèrement augmentée.

## TABLEAU III

| Echantillon | Cytotoxicité | | Vitesse |
|---|---|---|---|
| | Directe | Indirecte | de croissance cellulaire |
| témoin sans derme artificiel | / | / | Normale |
| Collagène H + Ch + GAG stérilisé à l'alcool | Négative | Négative | Normale |
| Collagène H + Ch + GAG stérilisé aux Rayons Gamma | Négative | Négative | Normale |
| Collagène B + Ch + GAG stérilisé à l'alcool | Négative | Négative | Normale |
| Collagène B + Ch + GAG stérilisé aux rayons Gamma | Négative | Négative | Normale |

### Etude de la biocompatibilité du derme artificiel

Les dermes artificiels stériles sont rincés dans deux bains de sérum physiologique et découpés aux dimensions désirées à l'aide de matériel chirurgical stérile.

L'animal choisi (rat blanc de laboratoire) est anesthésié par exposition à un mélange d'oxygène et d'halothane.

Il est rasé sur le dos et lavé avec une solution antiseptique. Deux excisions de 2 cm² sont pratiquées jusqu'au muscle sur le dos de l'animal avec un minimum de saignement. L'une reçoit du derme artificiel, l'autre sert de témoin.

Un bourdonnet est suturé sur chaque plaie et le tout est recouvert d'un pansement.

Plusieurs séries de trois rats sont réalisées sur ce modèle.

Les animaux sont sacrifiés à j 2, j 7, j 15, j 21 et j 30.

L'aspect du derme artificiel est alors noté, une biopsie est prélevée jusqu'au muscle dans les deux plaies pour l'étude en microscopie électronique à balayage et à transmission ainsi que pour l'étude histologique.

Le sang de l'animal est prélevé par ponction intracardiaque en vue des recherches d'anticorps.

Les résultats obtenus à j 2 montrent une réaction inflammatoire normale suivie à j 7 d'une colonisation cellulaire du derme artificiel. A j 15, aucune surinfection n'a été observée.

Les premiers résultats obtenus à j 21 montrent que le derme artificiel est toujours présent et est bien colonisé par les cellules.

Le derme artificiel ainsi obtenu peut être épidermisé en un second temps à l'aide d'un pseudo-épiderme biodégradable ; selon l'invention on utilise de préférence un pseudo-épiderme à base de chitosan seul ou associé à des chondroïtines sulfate et/ou à du collagène, ainsi qu'éventuellement à des protéines de structure modifiée ou à d'autres polyélectrolytes.

Une certaine lipophilie est avantageusement conférée au pseudo-épiderme par greffage chimique ou par application superficielle d'un succédané de sébum.

Le matériau composite derme + pseudo-épiderme ainsi réalisé présente tous les avantages énumérés ci-avant. Il est de plus entièrement biodégradable et pourra servir de support pour la culture des propres kératinocytes du sujet et être implanté dans les jours suivants les brûlures.

Relais précieux dans les techniques d'excision précoce du tissu brûlé au 3ème degré, il permet un recouvrement immédiat, évite les pertes exudatives et calorico-azotées, présente une barrière aux infections, limite les cicatrices hypertrophiques et les séquelles rétractiques invalidantes. Il est de plus un excellent support permettant d'envisage la culture de cellules épidermiques sur le malade lui-même.

Ainsi la reconstitution de la couche épidermique se fera en même temps que celle de la couche dermique, solution attendue par tous les Centres de Brûlés.

## EXEMPLE 2 : REALISATION D'UNE MATRICE EXTRACELLULAIRE FAVORISANT LA CROISSANCE DES CELLULES NERVEUSES

On réalise une solution à 1% (P/V) de collagène dans de l'acide acétique 0,05 N grâce à un agitateur vertical. Quand la solution est bien homogène, on dissout 0,15% de chitosan, puis 0,06% de glycosaminoglycanes.

La solution obtenue, qui possède les mêmes concentrations que le derme artificiel est coulée dans des tubes de silicones biocompatibles de diamètre variant entre 2 et 6 mm. Ces tubes sont lyophilisés puis stérilisés à l'alcool à 70° ou aux rayons gammas.

Ces tubes sont, dans un premier temps, prévus pour être implantés chez le lapin afin de tester les possibilités de régénération du nerf sciatique.

## EXEMPLE 3 : REALISATION D'UNE MATRICE EXTRACELLULAIRE POUR LA REGENERATION DE TISSU OSSEUX

On réalise une solution à 1% (P/V) de chitosan dans de l'acide acétique 0,5 N suivant la méthode décrite dans l'exemple 1. On ajoute à cette solution 16% de collagène par rapport au poids initial de chitosan, puis 84% de glycosaminoglycanes (toujours par rapport au chitosan).

Il se produit un précipité qui est accentué en ramenant le pH à un seuil proche de la neutralité. Ce précipité fibreux est récupéré, pressé et placé dans des tubes de silicones biocompatibles de 0,9 à 1,5 cm de diamètre. Au centre de ces tubes, une lumière de 1 à 2 mm de diamètre est réalisée. Elle peut être laissée ainsi ou emplie d'une solution de collagène (1%), chitosan (0,15%), glycosaminoglycanes (0,06%), soit les concentrations du derme artificiel.

Les tubes de silicone sont ensuite séchés à l'étuve ou lyophilisés. Leur aspect est alors dur et fibreux. Ils sont stérilisés aux rayons γ ou à l'alcool à 70°. Ces tubes sont, dans un premier temps, prévus pour être implantés chez le lapin ou le chien pour étudier les possibilités de régénération et de réparation d'os ou de fragments d'os lésés.

Du calcium peut être rajouté au mélange initial de façon à se rapprocher plus encore de la structure de l'os normal.

## EXEMPLE 4 : UTILISATION DES MELANGES PRECEDEMMENT DECRITS POUR REALISER DES ENVELOPPES BIOCOMPATIBLES DE PROTHESES DEJA EXISTANTES

Les prothèses de silicone (prothèse mammaire, prothèse de menton...) ou de Dacron (ligaments) ne sont pas recolonisées par les cellules ou le sont très lentement. Ceci occasionne des risques de glissement plus ou moins importants des prothèses de silicone, notamment des prothèses mammaires.

Un matériau biocompatible enveloppant ces prothèses permettra la régénération d'un tissu qui empêcherait ces glissements et constituerait une excellente interface prothèse-tissus environnants.

### Réalisation d'enveloppes biocompatibles

Une solution de collagène, chitosan et glycosaminoglycanes est réalisée dans les conditions de l'exemple 3. Les proportions de chaque constituant varient suivant la forme et la taille de la prothèse à recouvrir. En moyenne, les proportions optimales sont : collagène 0,5% (P/V), chitosan 0,08% et glycosaminoglycanes 0,03%.

La solution obtenue est dégazée à la centrifugeuse (trois quarts d'heure à 3000 tours/minute) puis le liquide homogène obtenu est étalé sur toute la surface de la prothèse et mis à l'étuve à 30°C jusqu'au séchage complet.

La prothèse et son enveloppe sont alors stérilisées à l'alcool ou aux rayons gammas.

**EXEMPLE 5 : UTILISATION DES DIFFERENTES MATRICES EXTRACELLULAIRES DECRITES DANS LES EXEMPLES 1 A 3 COMME SUPPORT DE CULTURE CELLULAIRE POUR DES ESSAIS PHARMACOTOXICOLOGIQUES**

La solution décrite dans les exemples 1 à 3 est coulée dans des flacons, des boîtes de Pétri ou des plaques multipuits et séchée à l'étuve à 30°C. Le film formé adhère à la boîte et représente un excellent support de cultures cellulaires.

D'autre part, les matrices lyophilisées peuvent être utilisées in vitro permettant de réaliser des cultures tridimensionnelles de cellules (fibroblastes, keratinocytes, chondrocytes...).

Ces modèles de culture peuvent servir à l'étude pharmacotoxicologique de différentes molécules chimiques ou médicamenteuses.

Dans les industries pharmaceutiques, ces modèles sont de plus en plus utilisés car ils permettent de faire un screening de familles moléculaires et de réduire considérablement l'utilisation d'animaux de laboratoire.

## Revendications

1. Biomatériau, caractérisé en ce qu'il comprend un polyélectrolyte obtenu par liaison ionique entre un collagène, un chitosan acétylé avec un degré d'acétylation compris entre environ 10 et environ 40%, et au moins un glycosaminoglycane.

2. Biomatériau selon la revendication 1, caractérisé en ce que le chitosan provient de la N-désacétylation de chitine (poly-N-acétylglucosamine) microfibrillaire de carapaces de crevettes.

3. Biomatériau selon la revendication 1, caractérisé en ce que le chitosan présente un poids moléculaire compris entre 150 000 et 1 000 000.

4. Biomatériau selon la revendication 1, caractérisé en ce que le collagène est choisi parmi les collagènes type III+I, les collagènes d'origine humaine ou bovine, les collagènes natifs, les collagènes sans télopeptides, à structure hélicoïdale préservée.

5. Application des biomatériaux selon l'une des revendications 1 à 4 en orthopédie et chirurgie plastique.

6. Application des biomatériaux selon l'une des revendications 1 à 4 à la réalisation des matrices extracellulaires pour la régénération des cellules nerveuses.

7. Application des biomatériaux selon l'une des revendications 1 à 4 à la réalisation de matrices extracellulaires pour la régénération des os.

8. Application des biomatériaux selon l'une des revendications 1 à 4 comme support de cultures cellulaires.

9. Application des biomatériaux selon l'une des revendications 1 à 4 à la réalisation d'enveloppes biocompatibles.

10. Peau artificielle caractérisée en ce qu'elle comprend une couche dermique de type velours comportant un biomatériau selon l'une quelconque des revendications 1 à 4.

11. Peau artificielle selon la revendication 10, caractérisée en ce que le glycosaminoglycane est choisi parmi les chondroïtines 4- et 6-sulfate et l'acide hyaluronique.

12. Peau artificielle selon les revendications 10 et 11, caractérisée en ce que la couche dermique comporte entre 10 et 20% de chitosan, entre 4 et 10% de chondroïtines 4- et 6-sulfate, ces pourcentages étant calculés par rapport au poids du collagène.

13. Peau artificielle selon l'une quelconque des revendications 10 à 12, caractérisée en ce que la couche dermique comporte en outre 1% en poids d'acide hyaluronique, par rapport au collagène.

14. Peau artificielle selon l'une quelconque des revendications 10 à 13, caractérisée en ce que la couche dermique est associée à un pseudo-épiderme biodégradable à structure membranée.

15. Peau artificielle selon la revendication 14, caractérisée en ce que le pseudo-épiderme est un film de chitosan.

16. Peau artificielle selon la revendication 14 et la revendication 15, caractérisée en ce que le film de chitosan est associé à des chondroïtines sulfate et/ou à du collagène.

17. Peau artificielle selon les revendications 15 et 16, caractérisée en ce que les composants du pseudo-épiderme sont associés à des protéines.

18. Peau artificielle selon l'une quelconque des revendications 14 à 17, caractérisée en ce que la lipophilie du pseudo-épiderme est modifiée par greffage chimique de substituants non toxiques hydrophobes, ou par application superficielle d'un succédané de sébum.

19. Procédé d'obtention d'une couche dermique artificielle selon l'une quelconque des revendications 10 à 13, caractérisé en ce qu'il comporte les étapes suivantes :

- dissolution du collagène dans de l'acide acétique ;
- addition à la solution de collagène ainsi obtenue, de chitosan purifié ;
- addition à la solution collagène-chitosan d'un mélange de chondroïtines 4- et 6-sulfate, suivie éventuellement d'addition d'acide hyaluronique ;
- neutralisation à pH 6,5-7 ;
- lyophilisation ou transformation en film de la solution neutralisée ;
- stérilisation.

## Ansprüche

1. Biomaterial, dadurch gekennzeichnet, daß es einen Polyelektrolyten enthält, der durch eine ionische Bindung zwischen einem Kollagen, einem acetylierten Chitosan mit einem Acetylierungsgrad zwischen etwa 10 und etwa 40% und zumindest einem Glykosaminoglykan erhalten wurde.

2. Biomaterial nach Anspruch 1, dadurch gekennzeichnet, daß das Chitosan aus einer N-Desacetylierung von mikrofaserigem Chitin (poly-N-Acetylglykosamin) aus Krabbenpanzern entstammt.

3. Biomaterial nach Anspruch 1, dadurch gekennzeichnet, daß das Chitosan ein Molekulargewicht zwischen 150 000 und 1 000 000 aufweist.

4. Biomaterial nach Anspruch 1, dadurch gekennzeichnet, daß das Kollagen ausgewählt ist aus den Kollagenen Typ III+I, den menschlichen Kollagenen oder den Kollagenen des Rindes, den freien Kollagenen, den Kollagenen ohne Telopeptide mit erhaltener schraubenlinienförmiger Struktur.

5. Anwendung des Biomaterials nach einem der Ansprüche 1 bis 4 in der Orthopädie und der Plastischen Chirurgie.

6. Anwendung des Biomaterials nach einem der Ansprüche 1 bis 4 zum Herstellen von extrazellulären Matrices zur Neubildung von Nervenzellen.

7. Anwendung des Biomaterials nach einem der Ansprüche 1 bis 4 zur Herstellung von extrazellulären Matrices zur Neubildung von Knochen.

8. Anwendung des Biomaterials nach einem der Ansprüche 1 bis 4 als Träger von Zellkulturen.

9. Anwendung des Biomaterials nach einem der Ansprüche 1 bis 4 zur Herstellung von biokompatiblen Umhüllungen.

10. Künstliche Haut, dadurch gekennzeichnet, daß sie eine samtartige Hautschicht aufweist, die ein Biomaterial nach einem der Ansprüche 1 bis 4 enthält.

11. Künstliche Haut nach Anspruch 10, dadurch gekennzeichnet, daß das Glykosaminoglykan ausgewählt ist aus Chondroitin-4-sulfat, Chondroitin-6-sulfat und der Hyaluronsäure.

12. Künstliche Haut nach Anspruch 10 oder 11, dadurch gekennzeichnet, daß die Hautschicht zwischen 10 und 20% Chitosan, zwischen 4 und 10% der Chondroitin-4 und -6-sulfate enthält, wobei die Prozentangaben bezüglich des Gewichtes an Rollagen berechnet sind.

13. Künstliche Haut nach einem der Ansprüche 10 bis 12, dadurch gekennzeichnet, daß die Hautschicht, bezogen auf das Kollagen, darüberhinaus 1 Gew.-% an Hyaluronsäure enthält.

14. Künstliche Haut nach einem der Ansprüche 10 bis 13, dadurch gekennzeichnet, daß die Hautschicht mit einer biologisch abbaubaren Pseudoepidermis mit membranartiger Struktur assoziiert ist.

15. Künstliche Haut nach Anspruch 14, dadurch gekennzeichnet, daß die Pseudoepidermis eine dünne Schicht aus Chitosan ist.

16. Künstliche Haut nach Anspruch 14 und 15, dadurch gekennzeichnet, daß die dünne Schicht aus Chitosan mit den Chondroitin-Sulfaten und/oder mit dem Kollagen assoziiert ist

17. Künstliche Haut nach Anspruch 15 oder 16, dadurch gekennzeichnet, daß die Bestandteile der Pseudoepidermis mit Proteinen assoziiert sind.

18. Künstliche Haut nach einem der Ansprüche 14 bis 17, dadurch gekennzeichnet, daß die Lipophilie der Pseudoepidermis durch chemische Veredelung mit hydrophoben, nichttoxischen Substituenten, oder durch oberflächliche Anwendung eines Talgersatzes modifiziert ist.

19. Verfahren zum Herstellen einer künstlichen Hautschicht nach einem der Ansprüche 10 bis 13, dadurch gekennzeichnet, daß es folgende Schritte aufweist :

- Auflösen von Kollagen in Essigsäure ;
- Hinzufügen von gereinigtem Chitosan zu der so erhaltenen Kollagen-Lösung ;

– Hinzufügen einer Mischung an Chondroitin-4 und -6-sulfaten zur Kollagen-Chitosan-Lösung, sowie ggf. Hinzufügen von Hyaluronsäure ;
– Neutralisieren auf pH 6,5-7 ;
– Lipophilisieren oder Überführen der neutralisierten Lösung in eine dünne Schicht ;
– Sterilisieren.


## Claims

1. Biomaterial, characterised in that it comprises a polyelectrolyte obtained by ionic bonding between a collagen, an acetylated chitosan with a degree of acetylation between approximately 10 and approximately 40% and at least one glycosaminoglycan.

2. Biomaterial according to Claim 1, characterised in that the chitosan results from the N-deacetylation of microfibrillar chitin (poly-N-acetylglucosamine) of prawn shells.

3. Biomaterial according to Claim 1, characterised in that the chitosan possesses a molecular weight of between 150,000 and 1,000,000.

4. Biomaterial according to Claim 1, characterised in that the collagen is selected from type III+I collagens, collagens of human or bovine origin, native collagens and telopeptide-free collagens, having a helical structure preserved.

5. Application of the biomaterials according to one of Claims 1 to 4 in orthopaedics and plastic surgery.

6. Application of the biomaterials according to one of Claims 1 to 4 to the production of extracellular matrices for nerve cell regeneration.

7. Application of the biomaterials according to one of Claims 1 to 4 to the production of extracellular matrices for bone regeneration.

8. Application of the biomaterials according to one of Claims 1 to 4 as a support for cell cultures.

9. Application of the biomaterials according to one of Claims 1 to 4 to the production of biocompatible shroudings.

10. Artificial skin, characterised in that it comprises a dermal layer of the smooth type containing a biomaterial according to any one of Claims 1 to 4.

11. Artificial skin according to Claims 10, characterised in that the glycosaminoglycan is selected from chondroitin 4- and 6-sulphates and hyaluronic acid.

12. Artificial skin according to Claims 10 and 11, characterised in that the dermal layer contains between 10 and 20% of chitosan and between 4 and 10% of chondroitin 4- and 6-sulphates, these percentages being calculated relative to the weight of the collagen.

13. Artificial skin according to any one of Claims 10 to 12, characterised in that the dermal layer contains, in addition, 1% by weight of hyaluronic acid relative to the collagen.

14. Artificial skin according to any one of Claims 10 to 13, characterised in that the dermal layer is combined with a biodegradable pseudo-epidermis having a membranous structure.

15. Artificial skin according to Claim 14, characterised in that the pseudo-epidermis is a film of chitosan.

16. Artificial skin according to Claim 14 and Claim 15, characterised in that the film of chitosan is combined with chondroitin sulphates and/or with collagen.

17. Artificial skin according to Claims 15 and 16, characterised in that the components of the pseudo-epidermis are combined with proteins.

18. Artificial skin according to any one of Claims 14 to 17, characterised in that the lipophilicity of the pseudo-epidermis is modified by the chemical grafting of hydrophobic, non-toxic substituents, or by the superficial application of a sebum substitute.

19. Method for obtaining an artificial dermal layer according to any one of Claims 10 to 13, characterised in that it comprises the following steps :
– dissolution of collagen in acetic acid ;
– addition of purified chitosan to the collagen solution thereby obtained ;
– addition of a mixture of chondroitin 4- and 6-sulphates to the collagen/chitosan solution, optionally followed by addition of hyaluronic acid ;
– neutralisation to pH 6.5-7 ;
– lyophilisation of the neutralised solution or conversion of the latter to a film ; and
– sterilisation.

FIG.1

FIG. 2

FIG. 3

FIG.4

FIG.5